# EUROPÄISCHE PATENTANMELDUNG

(11) **EP 0 733 713 A1**
(43) Veröffentlichungstag der Anmeldung: **25.09.1996**
(21) Anmeldenummer: 96104101.9
(22) Anmeldetag: 15.03.1996
(51) Int. Cl.: C12Q 1/68

(54) **Verfahren zur Messung Transkriptions-regulatorischer Aktivitäten von DNS-Sequenzen**

(30) Priorität: 18.03.1995 DE 19509898
(71) Anmelder: BOEHRINGER MANNHEIM GMBH, 68298 Mannheim (DE)
(72) Erfinder: Abken, Hinrich Johann, Dr., 56414 Meudt-Dahlen (DE); Reifenrath-Biesel, Barbara, 23845 Grabau (DE)

(57) **Zusammenfassung**

Die Erfindung betrifft ein Verfahren zur Messung Transkriptions-regulatorischer Aktivitäten von DNS-Sequenzen mit Hilfe der RNS-Transkription eines Reporter-Konstrukts i*n vitro* in Gegenwart eines nukleären Extrakts und der quantitativen Bestimmung der synthetisierten Reporter-RNS.

## Beschreibung

Der Phänotyp einer Zelle wird durch die koordinierte Ausprägung einer Vielzahl von Genen aufrecht erhalten. Dieses erfordert ein komplexes Netzwerk zur Regulation der Genexpression, insbesondere der RNS Transkription der Gene. Bestimmte, funktionell definierte DNS-Sequenzen vermitteln die quantitative Modulation der RNS Expression eines Gens. Diese Transkriptions-regulatorischen DNS-Elemente werden in folgenden Gruppen klassifiziert: (a) Promotoren, die den Startpunkt der RNS Transkription umfassen und somit die RNS-Transkription eines Gens ermöglichen; (b) "Verstärker" (enhancer), die die Anzahl der generierten RNS Transkripte eines Gens pro Zeit erhöhen; (c) "Suppressor" (silencer), die die Aktivität des Promotors unterdrücken und die Anzahl der synthetisierten RNS Transkripte pro Zeit erniedrigen. Promotoren liegen stromaufwärts nahe der transkribierten DNS-Region und wirken ausschließlich *in cis*. Im Gegensatz dazu können enhancer und silencer Elemente sowohl stromaufwärts wie auch -abwärts des jeweiligen Gens liegen und über sehr lange Distanzen (mehrere kb) auf Promotoren wirken. Diese beeinflußten Promotoren können sowohl *in cis* auf demselben DNS-Molekül als auch *in trans* auf anderen DNS-Molekülen liegen. Die Transkriptions-modulierenden DNS-Elemente zeigen in ihrer DNS-Sequenz eine sehr große Varianz, so daß diese Elemente ausschließlich durch ihre Funktion definiert werden, die RNS Expression eines Gens quantitativ zu modulieren. Die Aktivität dieser DNS-Elemente ändert sich mit dem jeweiligen physiologischen Zustand und dem Differenzierungsgrad der Zelle.

Da kein gemeinsames DNS-Sequenz-Motiv mit Sicherheit eine DNS-Sequenz als Transkriptions-regulatorisches Element kennzeichnet, wird zur Erkennung der Transkriptions-regulatorischen Aktivität von DNS-Sequenzen bisher ein Verfahren verwendet (Gorman, C.M., Moffat, L.F., Howard, B.H., Mol. Cell. Biol. 2: 1044 - 1051, 1982), bei dem die zu prüfende DNS-Sequenz stromaufwärts eines Reporter-Gens (z. B. das Gen für die Chloramphenicolacetyltransferase [CAT] oder das Gen für die β-Galaktosidase [β-gal]) in ein Plasmid kloniert und das gewonnene rekombinante DNS-Konstrukt in eine Prüfzelle transferiert wird. In einem Kontrollexperiment wird ein Standardkonstrukt ohne dieses DNS-Element transfiziert. Zwei Tage nach Transfektion wird ein Lysat der Zellen hergestellt und das vom Reportergen kodierte Protein oder dessen enzymatische Aktivität in diesem Lysat bestimmt. Zur Messung der Reporter-Protein-Menge steht ein ELISA-Verfahren (Boehringer Mannheim) zur Verfügung, zur Bestimmung der enzymatischen Aktivität des Reporter-Proteins werden die enzymatischen Spaltprodukte eines radioaktiven Substrats mit Hilfe der Dünnschichtchromatographie aufgetrennt und quantitativ bestimmt (Gorman, C.M., Moffat, L.F., Howard, B.H., Mol. Cell. Biol. 2: 1044 - 1051, 1982). In Abhängigkeit von der Aktivität des Transkriptions-regulatorischen DNS-Elements werden verschieden große Mengen Reporter-Protein in der Zelle nach Transfektion synthetisiert. Der Vergleich mit dem Kontrollexperiment gibt Auskunft darüber, ob die Anwesenheit des DNS-Elements die synthetisierte Menge Reporter-Protein im Vergleich zum Standardkonstrukt erhöht (somit als Promotor, Enhancer wirkt) oder erniedrigt (Silencer).

Dieses bisher verwendete Verfahren geht von der Annahme aus, 1. daß die Menge synthetisierten Reporter-Proteins proportional der Menge mRNS des Reportergens und somit ein Maß für die Transkriptions-regulatorische Aktivität eines DNS-Elements ist, und 2. daß das synthetisierte Reporter-Protein in der Zelle ohne Degradation akkumuliert. Bei diesen Annahmen bleibt unberücksichtigt, daß folgende Prozesse das Meßergebnis erheblich beeinflussen: 1. quantitative Änderungen der zellulären Protein-Translation, die unabhängig von der RNS-Transkription des Reportergens und damit der Aktivität des DNS-Elements sind; 2. Degradierung des synthetisierten Reporter-Proteins durch zelluläre Proteasen; 3. bei der Bestimmung der Menge des Reporter-Proteins durch Messung von dessen enzymatischen Aktivität können zelluläre Enzyme interferieren.

Weiterhin setzt die Durchführbarkeit des bisher verwendeten Verfahrens voraus, daß ein effizienter Transfer des Reporter-DNS-Konstrukts in die Prüfzelle erfolgt. Die Transfektionseffizienz ist jedoch von dem verwendeten Transfektionsverfahren, der jeweiligen Prüfzelle und von ihrem physiologischen Zustand in erheblichem Maße abhängig. Außerdem ist die Transfektionseffizienz einer hohen Varianz unterworfen. Da jedoch verschieden hohe Transfektionseffizienzen fälschlich verschieden hohe Aktivitäten der DNS-Elemente wiederspiegeln, müssen die Meßwerte stets auf die tatsächlich in die Zelle übertragene DNS normiert werden, was eine vergleichende Bestimmung der in der Zelle übertragenen Menge Reporter-DNS in jedem Transfektionsansatz notwendig macht. Dieses kann z. B. mit Hilfe von Hybridisierungsverfahren erfolgen (Abken und Reifenrath, Nucl. Acids Res. 20: 3527, 1992). Der Einschluß dieser notwendigen Kontrollen macht jedoch das bisherige Meßverfahren zur Bestimmung der Transkriptions-regulatorischen Aktivität von DNS-Elementen extrem aufwendig.

Eine weitere Einschränkung in der Anwendung dieses Verfahrens ergibt sich dadurch, daß der derzeitige Stand der Technik eine Transfektion nur weniger Zell-Linien mit hinreichend hoher Effizienz erlaubt. Aus diesem Grund werden die Analysen Transkriptions-regulatorischer Aktivität von DNS-Sequenzen auf Tumorzell-Klone mit sehr guter Transfektionseffizienz beschränkt Zellen mit extrem geringer Transfektionseffizienz, z. B. primäre Leberzellen, Lymphozyten aus dem peripheren Blut oder neutronale Zellen, konnten bisher kaum als Prüfzellen eingesetzt werden, was zur Folge hat, daß die Aktivität Transkriptions-regulatorischer DNS-Elemente in zahlreichen normalen Zellen terminalen Differenzierungsgrades unbekannt ist.

Aufgabe der vorliegenden Erfindung war es daher, ein Verfahren zur Verfügung zu stellen, bei dem die Aktivität Transkriptions-regulatorischer DNS-Elemente unabhängig von DNS-Transfektionen in Prüfzellen und unabhängig von post-transkriptionellen Modifikationen des Reporter-Produkts gemessen werden kann.

Gelöst wird die Aufgabe dadurch, daß ein Reporter-DNS-Konstrukt in Gegenwart eines nukleären Extraktes in vitro in RNS transkribiert und die Menge der Reporter-RNS im Vergleich mit der eines Referenz-DNS-Konstrukts bestimmt wird.

Ausgangspunkt des erfindungsgemäßen Verfahrens ist die Beobachtung, daß kodierende DNS-Sequenzen in vitro unabhängig von der intakten Zellstruktur in Gegenwart eines nukleären Extraktes in RNS transkribiert werden können (Dignan, J.B., Lebovitz, R.M., Roeder, R.G., Nucl. Acids Res. 11: 1475 - 1489, 1983). Wider Erwarten hat sich nun gezeigt, daß sich diese Eigenschaft in der Anwendung auf ein rekombinantes Reporter-DNS-Konstrukt nutzen läßt. Das Konstrukt trägt dabei das zu prüfende Transkritions-regulatorische DNS-Element sowie ein Reporter-Gen. Als Reporter-Gen kann jede beliebige für ein bestimmtes Peptid oder Protein kodierende DNS- oder cDNS-Sequenz oder eine artifizielle synthetische DNS-Sequenz oder jeweils Fragmente davon dienen, die in dem Genom der Prüfzelle nicht vorkommen. Die Zellen können eukaryotischer oder prokaryotischer Natur sein. Typischerweise wird das Transkripitons-regulatorische DNS-Element zur Prüfung auf Promotor-Aktivität stromaufwärts des Reporroter-Gens in das Reporter-DNS-Konstrukt inseriert. Da Enhancer und Silencer bidirektional wirken, können diese DNS-Elemente sowohl stromaufwärts als auch stromabwärts von der Reporter-Expressionskassette inseriert werden.

Der nukleäre Extrakt wird erfindungsgemäß aus den Prüfzellen gewonnen, in deren Gegenwart die Aktivität Transkriptions-regulatorischer DNS-Elemente bestimmt werden soll. Bevorzugt wird als Reporter-Gen das Gen der Chloramphenicolacetyltransferase (CAT) verwendet. Aber auch das Gen für β-Galactosidase und das Gen für Luciferase haben sich als geeignet erwiesen. Insbesondere werden Zellen etablierter Tumor-Linien, z. B. Zellen der menschlichen B-Lymphom-Linie BJAB, oder auch primäre Zellen, z. B. Lymphozyten aus dem peripheren Blut, oder auch Gewebe und Biopsien verwendet.

Der nukleäre Extrakt wird nach dem Fachmann bekannten Verfahren präpariert (Dignan, J.D., Lebovitz, R.M., Roeder, R.G., Nucl. Acids Res. 11: 1475 - 1489, 1983). Vorzugsweise werden die Zellen in Gegenwart von Tris (50 mM), pH 7,6, EDTA (10mM) und MgCl₂ (3mM) durch wiederholtes Einfrieren und Auftauen lysiert. Alternativ kann die Zell-Lyse in Gegenwart von Detergentien, wie z. B. SDS und NP40, erfolgen. Die Zellkerne werden von Fragmenten des Zytoplasmas durch Zentrifugation abgetrennt und anschließend lysiert, bevorzugt durch Homogenisieren in einem Glashomogenisator. Nach Abtrennung von Membranen und größeren Fragmenten durch Zentrifugation wird der Überstand dialysiert, bevorzugt gegen einen Puffer aus 20 mM Hepes, pH 7.9, 100 mM KCL, 0,2 mM EDTA, 0,5 mM PMSF, 0,5 mM DTT, 20% vol/vol Glycerin. Der Kernextrakt wird nochmal zentrifugiert und schließlich bei -70°C gelagert. Der Extrakt bewahrt seine Aktivität für die Anwendung in dem erfingungsgemäßen Verfahren bei dieser Lagerung mindestens 3 Monate.

Erfindungsgemäß wird das Reporter-DNS-Konstrukt in Gegenwart des nukleären Extrakts in vitro transkribiert. Die in vitro Transkription erfolgt nach dem Fachmann bekanntem Verfahren (z. B. Dignan, J.D., Lebovitz, R.M., Roeder, R.G., Nucl. Acids Res. 11: 1475 - 1489, 1983; Müller, H.-P., Sogo, J.M., Schaffner, W., Cell 58: 767 - 777, 1989). Bevorzugt ist hier folgendes Verfahren: Die rekombinante Reporter-DNS wird zunächst in Reaktionspuffer (1 mM Hepes, pH 7,9, 2mM KCl, 0,7 mM NaCl, 0,5 mM Mg-acetat, 0,01 mM EDTA, 0,85% vol/vol Glycerin, 1 mM DTT, 10 mM Phosphocreatin, 1 U human Plazenta RNase Inhibitor (RNasin) (Boehringer Mannheim), je 1mM ATP, TTP, CTP und GTP) in Gegenwart des nukleären Extraktes (entsprechend Extrakt aus 10⁶ Zellkernen) aus der Prüfzelle 1 Std. bei 30°C zur Transkription der Reporter-RNS inkubiert. Die Menge der transkribierten Reporter-RNS ist abhängig von der Länge der Inkubationszeit der in vitro Transkription sowie der Menge Reporter-DNS und nukleären Extrakt pro Ansatz. Quantitative Vergleiche paralleler Ansätze erfordern deswegen konstante Inkubationszeiten der *in vitro* Transkription, ca. 20 bis 90 Min., bevorzugt 1 Std., äquimolare Menge an Reporter-DNS (bevorzugt 1pMol DNS) und konstante Menge an nukleärem Extrakt, bevorzugt aus 10⁶ Zellen; pro Ansatz.

Zum spezifischen Nachweis der transkribierten Reporter-RNS bieten sich Verfahren der Nukleisäure-Hybridisierung an, insbesondere die Hybridisierung der RNS mit einer markierten DNS- oder RNS-Probe oder die Hybridisierung der während der in-vitro-Transkription markierten RNS an eine unmarkierte DNS- oder RNS-Probe. Erfindungsgemäß wird das Verfahren der RNS dot blot Hybridiesierung bevorzugt. Dabei wird wie folgt vorgegangen: Die in dem Transkripitonsansatz befindliche DNS wird enzymatisch abgebaut, die RNS wird denaturiert und der Ansatz auf eine Träger-Membran (z. B. Nylon-Membran) übertragen. Die Membran wird mit einer markierten DNS-Probe, die homolog zu der Reporter-DNS Sequenz ist, hybri-disiert. So findet z. B. bei Verwendung des Gens der Chlor-amphenicolacetyltransferase (CAT) als Reproter-Gen bevorzugt die mit Digoxigenin-(DIG-)markierte CAT DNS als Hybridisierungsprobe Anwendung. Die Intensität des Hybridisierungssignals gibt ein Maß für die Menge der synthetisierten Reporter-RNS im Vergleich zu dem Signal eines geeigneten Kontrolansatzes und erlaubt somit die vergleichenede Messung der Aktivität Transkriptions-regulatorischer DNS-Elemente.

Eine weitere Ausführungsform des erfindungsgemäßen Verfahrens ist, daß die Reporter-RNS durch Hybridisierung an eine Fangprobe und einen anschließenden ELISA nachgewiesen wird. In dieser Durchführung wird in Abwandlung des oben beschriebenen Verfahrens die in vitro Transkription in Gegenwart eines modifizierten Nukleotides, z. B. Digoxigenin-UTP (DIG-UTP) (Boehringer Mannheim), durchgeführt. Dabei wird die transkribierte Reporter-RNS mit DIG-UTP markiert. Als Fangprobe zur spezifischen Bindung der Reporter-RNS wird ein Oligonukleotid bevorzugt, das homolog zu der kodierenden Reporter DNS Sequenz ist. Das Oligonukleotid ist mit einem weiteren modifizierten Nukleotid markiert, z. B. Biotin-dUTP. Der Transkripitonsansatz wird denaturiert und anschließend mit dem Fangoligonukleotid hybridisiert. Die (hybridisierten und freien) Fangoligonukleotide werden an einen Träger gebunden, z. B. über eine Biotin-Streptavidin-Brücke an die Wand einer Mikrotiterplatte. Nach mehrfachem Waschen des Trägers wird ein ELISA zur Bestimmung der in die Reporter-RNS eingebauten modifizierten Nukleotide (z. B. DIG-UTP) unter Verwendung eines spezifischen Antikörpers (z. B. anti-DIG-Antikörper) nach dem Fachmann bekannten Verfahren durchgeführt. Das Meßsignal ist der in die Reporter-RNS eingebauten modifizierten Nukleotide und der Menge der synthetisierten Reporter-RNS proportional und im Vergleich mit einem geeigneten Kontrollkonstukt ein Maß für die Transkriptions-regulatorische Aktivität der DNS-Elemente.

Alternativ kann die *in vitro* in Gegenwart eines modifizierten oder radioaktiven Nukleotids synthetisierte Reporter-RNS durch Auftrennung in einem denaturierenden Polyacrylamid-Gel nachgewiesen und durch Densitometrie oder Scintillationsspektrometrie nach bekannten Verfahren quanitfiziert werden (Phillips, D.R., und Crothers, D.M., Biochemistry 25: 7355 - 7362, 1986). In einer weiteren Abwandlung des erfindungsgemäßen Verfahrens kann die *in vitro* synthetisierte Reporter-RNS durch Hybridisierung mit einer spezifischen DNS-Probe und anschließendem S1-Nuklease-Abbau nach bekanntem Verfahren nachgewiesen werden ("S1-Analyse") (Sambrook, Fritsch, Maniatis: Molecular Cloning, 2nd edition, Cold Spring Harbor Laboratories, Cold Spring Habor, 1989).

Ein weiterer Gegenstand der Erfindung ist ein Reagenz zur Bestimmung der Aktivität Transkriptorischer DNS-Sequenzen, welches eine im pH-Bereich zwischen 6,5 bis 8,2 puffernde Substanz, die für die Transkription erforderlichen Salze und Zusätze, einen RNase-Inhibitor und ausreichende Mengen an ATP, TTP, CTP und GTP enthält. Insbesondere haben sich hier sogenannte GOOD-Puffer, vorzugsweise in einer Konzentration von 0,1 bis 10 mM, als geeignet erwiesen.

Das erfindungsgemäße Verfahren besitzt somit folgende Vorteile:
(1) Die Identifizierung von Transkriptions-regulatorischen Aktivitäten von DNS-Sequenzen, deren biologische Aktivität bisher unbekannt ist, ist ohne besonderen Zeit- und Apparateaufwand möglich. Des weiteren sind
(2) die quantitativ-vergleichende Messung der Stärke verscheidener Transkriptions-regulatorischer DNS-Elemente, z. B. Promotoren, Enhancer und Silencer, in Gegenwart eines nukleären Extrakts eines Zelltyps,
(3) die quantitativ-vergleichende Messung der Stärke eines Transkriptions-regulatorischen DNS-Elements in Gegenwart nukleärer Extrakte verschiedener Zellen,
(4) die quantitativ-vergleichende Messung der Stärke eines Transkriptions-regulatorischen DNS-Elements bezüglich seiner Wirkung auf die RNS-Transkripiton der Reporter-DNS stromaufwärts oder stromabwärts desselben DNS-Moleküls (Wirkung in cis) und bezüglich seiner Wirkung auf die RNS-Transkription der Reporter-DNS auf einem separaten DNS-Molekül (Wirkung in trans), möglich.

Die Erfindung wird durch folgende Beispiele näher erläutert.

### Beispiel 1

### Identifizierung einer bisher unbekannten Transkriptions-regulatorischen Aktivität der DNS-Sequenzen EFC38.

Die DNS-Sequenz EFC38 (372 bp) wurde von Abken et al., 1993 (Proc. Natl. Acad. Sci. USA 90: 6518 - 6522, 1993) aus Tumorzellen kloniert. Die DNS kodiert nicht für ein Protein. Die biologische Aktivität des DNS-Elements ist bisher unbekannt. Aufgabe ist es, mögliche Transkriptions-regulatortische Aktivitäten der EFC38 DNS zu identifizieren.
(a) Präparation des nukleären Extraktes.
Der nukleäre Extrakt wird nach dem Fachmann bekannten Verfahren aus Zellen der menschlichen Lymphom-Linie BJAB gewonnen (Dignan, J.D., Lebovitz, R.M., Roeder, R.G., Nucl. Acids Res. 11: 1475 - 1489, 1983). Ca. 10⁹ BJAB-Zellen werden in Gegenwart von Tris (50mM), pH 7.6, EDTA (10mM) und MgCl₂ (3mM) durch wiederholtes Einfrieren und Auftauen lysiert. Die Zellkerne werden durch Zentrifugation bei 8000 g, 5 Min, 4°C sedimentiert und in 30 ml Hepes (20 mM), pH 7.9, MgCL₂ (1,5 mM), NaCL (420 mM), DTT (0,5 mM), EDTA (0.2 mM), PMSF (0.5 mM) und Glycerin (25% vol/vol) resuspendiert. Die Zellkerne werden durch Homogenisieren in einem Glashomogenisator lysiert. Das Lysat wird 30 Min. bei 4°C gerührt. Anschließend werden die Membranen und größere Fragmene durch Zentrifugation bei 25.000 g, 4°C für 30 Min. sedimentiert. Der Überstand wird gegen das 50fache Volumen des Dialysepuffers (20 mM Hepes, pH 7.9, 100 mM KCL, 0.2 mM EDTA, 0.5 mM PMSF, 0,5 mM DTT, 20% vol/vol Glycerin) übernachtet bei 4°C dialysiert. Der Kernextrakt wird nochmals bei 25.000 g, 4°C für 20 Min. zentrifugiert und schließlich bei -70°C gelagert.
(b) Rekombinante DNS-Konstrukte.
Das Plasmid pCAT enthält die DNS-Sequenz des Reporter-Gens, das für die Chloramphenicolacetyltransferase kodiert, ohne Promotor, Enhancer oder Silencer (Gorman, C.M., Moffat, L.F., Howard, B.H., Mol. Cell. Biol. 2: 1044 - 1051, 1982). Das Plasmid pfos-CAT enthält den menschlichen c-fos Promotor stromaufwärts der kodierenden CAT Sequenz (Gilman, M.Z.,Wilson, R.N., Weinberg, R.A., Mol. Cell, Biol. 6: 4305 - 4316, 1986). Zur Prüfung auf Enhancer-Silencer-Aktivität wird die EFC38 DNS stromaufwärts des c-fos Promotors in das Plasmid pfos-CAT kloniert (pEFC38-fos-CAT).
(c) *In vitro* Transkription
Die o.g. DNS-Konstrukte wurden der *in vitro* Transkription unterworfen. Als weitere Kontrolle diente ein Ansatz ohne DNS-Konstrukt ("Leerwert"). Die *in vitro* Transkription der Reporter-Konstrukte erfolgt in Gegenwart des nukleären Extrakts aus BJAB-Zellen. Je 1 µg der rekombinanten Reporter DNS wird in 15 µl Reaktionspuffer (1mM Hepes, pH 7.9, 2 mM KCL, 0.7 mM NaCl, 0.5 mM Mgacetat, 0.01 mM EDTA, 0,85% vol/vol Glycerin, 1 mM DTT, 10 mM Phosphocreatin, 1 U human Plazenta RNase Inhibitor (RNasin) (Boehringer Mannheim), je 1 mM ATP, TTP, CTP und GTP) in Gegenwart von 5 µl des nukleären BJAB-Extrakts (entsprechend Extrakt aus 10⁶ Zellkernen) 1 Std. bei 30°C zur Transkription der Reporter-RNS inkubiert.
(d) Nachweis der synthetisierten CAT RNS.
Die DNS in dem Transkriptionsansatz wird durch Inkubation mit 10 U DNase (RNase-frei) für 10 Min. bei 37°C abgebaut. Zum Reaktionsansatz werden 200 µl 20xSSC hinzugegeben, und die RNS wird durch Inkubation bei 90°C für 1 Min. denaturiert. Der Ansatz wird in Vedünnungen (z. B. 90 µl, 30 µl, 10 µl, 3 µl, 1 µl) auf eine Träger-Membran (z. B. Nylon-Membran) übertragen. Die Membran wird mit einer Digoxigenin-dUTP markierten CAT DNS-Probe (1 kb), die homolog zu der kodierenden DNS Sequenz des Reporter-Gens ist, hybridisiert. Das Autoradiogramm wird densitometrisch vermessen. Die Intensität des Hybridisierungssignals gibt ein Maß für die Menge der synthetisierten Reporter-RNS im Vergleich zu dem Signal des Kontrollansatzes mit dem Plasmid pCAT (für die Bestimmung der Promotor-Aktivität) bzw. pfos-CAT (für die Bestimmung der Enhancer-/Silencer-Aktivität) (Tabelle 1).

**Tabelle 1**

| **Bestimmung der Transkriptions-regulatorischen Aktivität des DNS-Elements EFC38.** | | |
|---|---|---|
| DNS-Konstrukt | densitometrische Einheit [dU] | Faktor [f] |
| -- | 100 | < 0,01 |
| pCAT | 2.473 | 0,23 |
| pfos-CAT | 10.762 | 1,0 |
| pEFC38-fos-CAT | 4.408 | 0,41 |

(e) Ergebnis:
Das DNS-Element EFC38 hat Silencer-Aktivität, da die Aktivität des menschlichen c-fos Promotors durch die Anwesenheit des EFC38 DNS-Elements in dem Reporter-Konstrukt pEFC38-fos-CAT supprimiert wird.

### Beispiel 2

### Quantitativer Vergleich der Transkriptions-regulatorischen Aktivität verschiedener Promotor/Enhancer Elemente.

Folgende Promotor/Enhancer-DNS Elemente sollen in ihrer Transkripitons-regulatorischen Aktivität verglichen werden:
- c-fos Promotor: aus dem menschlichen Genom.
- HCMV-LTR: aus menschlichem Cytomegalovirus.
- MPSV-LTR: aus
- RSV-LTR: aus Rous-Sarcom-Virus.

Diese DNS Elemente wurden jeweils stromaufwärts in das Plasmid pCAT kloniert: pfos-CAT, pHCMV-CAT, pMPSV-CAT, pRSV-CAT. Jeweils 1 µg der rekombinanten DNS wurde wie in Beispiel 1 (c) beschrieben der *in vitro* Transkription in Gegenwart von nukleärem Extrakt aus BJAB-Zellen unterworfen. Der Ansatz wurde denaturiert, in Verdünnungen auf eine Nylon-Membran aufgetragen und die synthetisierte CAT RNS durch Hybridisierung mit der DIG-markierten CAT DNS Probe wie in Beispiel 1 (d) beschrieben nachgewiesen und das Autoradiogramm densitometrisch ausgewertet. Zum Vergleich der Promotor/Enhancer-Aktivitäten wurde der Probe mit der geringsten Menge CAT mRNS der Faktor 1 zugeordnet.

**Tabelle 2**

| Vergleich der Transkriptions-regulatorischen Aktivitäten verschiedener DNS-Elemente. | | |
|---|---|---|
| DNS-Konstrukt | densitometrische Einheit [dU] | Faktor [f] |
| -- | 480 | < 0,03 |
| pfos-CAT | 18.250 | 1 |
| pHCMV-CAT | 129.390 | 7 |
| pMPSV-CAT | 137.900 | 7,5 |
| pRSV-CAT | 44.620 | 2,4 |

### Ergebnis:

In Gegenwart des nukleären Extrakts aus den menschlichen BJAB-Lymphom-Zellen zeigen die geprüften DNS-Elemente verschieden starke Promotor/Enhancer-Aktivitäten, und zwar MPSV LTR > HCMV LTR > RSV LTR > fos-Promotor.

### Beispiel 3

### Vergleich der Transkripitons-regulatorischen Aktivität des menschlichen c-fos Promotors in Gegenwart verschiedener nukleärer Extrakte.

Die Aktivitäten des menschlichen c-fos Promotors sollen in Gegenwart von nukleären Extrakten aus der menschlichen Lymphom-Linie BJAB (tumorigenen Ursprungs) und der menschlichen lymphoiden Linie 654 (nicht-tumorigenen Ursprungs) verglichen werden.

Die nukleären Extrakte aus jeweils 10⁹ Zellen der Linien BJAB und 654 wurden wie in Beispiel 1 (a) beschrieben hergestellt. Der menschliche c-fos Promotor wurde stromaufwärts der CAT DNS-Sequenz in das Plasmid pCAT inseriert (pfos-CAT). Die pfos-CAT DNS (1 µg) wurde in vitro in Gegenwart von BJAB- bzw. 654-Extrakt (jeweils Extrakt aus 10⁶ Zellen pro Ansatz) wie in Beispiel 1 (c) beschrieben transkribiert. Der Ansatz wurde auf eine Nylon-Membran übertragen und die synthetisierte CAT RNS durch Hybridisieren mit der DIG-markierten CAT DNS-Probe nachgewiesen. Das Autoradiogramm wurde densitometrisch vermessen (Tabelle 3).

**Tabelle 3**

| **Vergleich der Transkriptions-regulatorischen Aktivität des c-fos Promotors in Gegenwart verschiedener nukleärer Extrakte.** | | | |
|---|---|---|---|
| DNS-Konstrukt | nukleärer Extrakt aus | densitometrische Einheit [dU] | Faktor [f] |
| pfos-CAT | BJAB-Zellen | 6.721 | 0,8 |
| pfos-CAT | 654-Zellen | 8.487 | 1,0 |

| Kontrollen: | | | |
|---|---|---|---|
| -- | BJAB-Zellen | 85 | < 0,01 |
| -- | 654-Zellen | 115 | < 0,18 |

### Ergebnis:

Die Transkriptions-regulatorische Aktivität des menschlichen c-fos Promotors ist in Gegenwart des nukleären Extrakts aus der Lymphom-Linie BJAB um den Faktor - als in Gegenwart des Extrakts aus der nicht-tumorigenen Linie 654.

### Beispiel 4

### Bestimmung der Transkriptions-regulatorischen Aktivität des EFC38 DNS-Elements in cis und in trans.

Es soll geprüft werden, ob die Silencer-Aktivität des DNS-Elements EFC38 ausschließlich *in cis* stromaufwärts oder stromabwärts auf den menschlichen c-fos Promotor desselben DNS-Moleküls wirkt oder auch *in trans*, wenn sich der c-fos Promotor auf einem separaten DNS-Molekül befindet.

Das EFC38 DNS-Element (372 bp) wird stromaufwärts des c-fos Promotors in das Plasmid pfos-CAT inseriert (pEFC38-fos-CAT). Dieses rekombinante Konstrukt wird in Gegenwart des nukleären Extrakts von BJAB-Zellen in vitro transkribiert (Wirkung *in cis*).

Weiterhin werden Proteine aus Ehrlich Aszites Zellen an das EFC38 DNS Element gebunden, die die mögliche trans-Aktivität vermitteln sollen. Diese Proteine zeigen selbst keine Transkriptions-regulatorische Aktivität. Anschließend wird die Silencer-Aktivität des EFC38 DNS-Element *in trans* auf das separate pfos-CAT DNS-Molekül gemessen (Wirkung *in trans*). Dazu wird die Protein-beladene EFC38 DNS zusammen mit der pfos-CAT DNS einer in vitro Transkription in Gegenwart des nukleären Extrakts aus BJAB-Zellen wie in Beispiel 1 (c) beschrieben unterworfen.

Die synthetisierte CAT RNS wird jeweils durch Hybridisierung mit einer DIG-markierten CAT DNS Probe nachgewiesen und das Autoradiogramm densitometrisch ausgewertet (Tabelle 4).

**Tabelle 4**

| **Silencer-Aktivität des EFC38 DNS-Elements in cis und in trans auf den c-fos Promotor.** | | |
|---|---|---|
| DNS-Konstrukt | densitometrische Einheit [dU] | Faktor [f] |
| -- | 100 | < 0,01 |
| pfos-CAT | 10.762 | 1,0 |
| pEFC38-fos-CAT | 95 | < 0,01 |
| EFC38 + pfos-CAT | 1.999 | 0,18 |

### Ergebnis:

Das EFC38 DNS-Element wirkt als Silencer sowohl *in cis* als auch *in trans* auf den menschlichen c-fos Promotor. Die Wirkung *in cis* ist um den Faktor 18 stärker als die Wirkung in trans.

## Patentansprüche

1. Verfahren zur Bestimmung der Aktivität Transkriptions- regulatorischer DNS-Sequenzen, dadurch gekennzeichnet,
daß man
(1) ein Reporter-DNS-Konstrukt in Gegenwart eines aus einer eukaryotischen oder prokaryotischen Zelle gewonnenen nukleären Extrakts in vitro in RNS transkribiert,
(2) die Menge der transkribierten Reporter-RNS bestimmt und
(3) aus dem Verhältnis von gebildeter Reporter-RNS zu der eines Referenz-DNS-Konstrukts die Transkriptions-regulatorische DNS-Sequenz identifiziert und dessen Aktivität quantifiziert wird.

2. Verfahren nach Anspruch 1, dadurch gekennzeichnet, daß das Reporter-DNS-Konstrukt aus einem Transkriptions-regulatorischen DNS-Element und einem heterologen Reporter-Gen besteht.

3. Verfahren nach Anspruch 2, dadurch gekennzeichnet daß das Reporter- Gen eine DNS, cDNS oder eine artifizielle synthetische DNS, welche für ein bestimmtes Petptid oder Protein kodiert, das eine enzymatische oder sonstige biologische Aktivität aufweist, oder ein geeignetes (synthetisches oder natürliches) DNS-Fragment darstellt.

4. Verfahren nach einem der Ansprüche 2 oder 3, dadurch gekennzeichnet, daß es sich bei dem Reporter-Gen um ein für Chlor-amphenicolacetyltransferase, β-Galaktosidase oder Luciferase kodierendes Gen handelt.

5. Verfahren nach Anspruch 2, dadurch gekennzeichnet, daß das Transkriptions-regulatorische DNS-Element stromaufwärts oder stromabwärts des Reporter-Gens in das Reporter-DNS-Konstrukt inseriert ist.

6. Verfahren nach Anspruch 1, dadurch gekennzeichnet, daß als Zellen etablierte menschliche oder tierische Zell-Linien oder primäre Zellen oder andere eukaryotische Zellen verwendet werden.

7. Verfahren nach einem der vorangegangenen Ansprüche, dadurch gekennzeichnet, daß ein aus Zellen gewonnener nukleärer Extrakt verwendet wird und die Transkription in einem geeigenten Reaktionspuffer in Gegenwart von für die Transkription erforderlichen Ribonuklotiden und gegebenenfalls eines modifizierten Ribonukleotides inkubiert wird.

8. Verfahren nach einem der vorangegangenen Ansprüche, dadurch gekennzeichnet, daß die gegebenenfalls markierte Reporter-RNS mit einer markierten oder nicht-markierten homologen RNS- oder DNS-Probe hybridiert wird oder mit einem gegen die Markierung der Reporter-RNS oder DNS- oder RNS- Probe gerichteten Antikörper in Kontakt gebracht und nachgewiesen wird.

9. Verfahren nach Anspruch 7, dadurch gekennzeichnet, daß in dem Ansatz nach Transkription vorhandene DNS enzymatisch abgebaut und abgetrennt, die RNS denaturiert wird und der Ansatz auf einen festen Träger gegeben wird und anschließend mit einer markierten, zur Reporter-Gen homologen DNS- Probe oder RNS-Probe hybridisiert wird.

10. Verfahen nach Anspruch 8 oder 9, dadurch gekennzeichnet, daß die Markierung mittels eines Biotin- und/oder Digoxigenin-Ribonukleotid oder - Desoxyribonucleotid durchgeführt wird.

11. Verfahren nach einem der vorangegangenen Ansprüche, dadurch gekennzeichnet, daß die Reporter-DNS das Gen für Chlor-amphenicolacetyltransferase (CAT), β-Galaktosidase (β-Gal) oder Luciferase enthält und die Menge an Transkriptions-regulatorischer DNS mittels Digoxigenin-markierter CAT, β-Gal oder Luciferase DNS erfolgt.
